# EUROPEAN PATENT APPLICATION

(11) **EP 4 775 083 A1**
(43) Date of publication of application: **15.07.2026**
(21) Application number: 24863236.6
(22) Date of filing: 06.09.2024
(51) Int. Cl.: A45D 40/00, B65D 83/00

(54) **CONTAINER**

(30) Priority: 06.09.2023 KR 20230118483
(71) Applicant: LG H&H CO., LTD., Seoul, 03184 (KR)
(72) Inventor: HAN, Jaesik, Seoul 07795 (KR); AN, Myoung Jin, Seoul 07795 (KR); LEE, In Sun, Seoul 07795 (KR); KIM, Kyung Won, Seoul 07795 (KR); WE, Jin Young, Seoul 07795 (KR); KIM, Eun Mi, Seoul 07795 (KR); LEE, Do Hoon, Incheon 21315 (KR)
(74) Representative: Vossius & Partner Patentanwälte Rechtsanwälte mbB
(86) International application number: PCT/KR2024/013456
(87) International publication number: WO 2025/053657

(57) **Abstract**

The present invention relates to a container according to an embodiment, the container comprising: a storage part for accommodating a content; a shaft part that rotates relative to the storage part; and a pressing part that is screw-coupled to the shaft part and pushes up the content due to the rotation of the shaft part, wherein the viscosity of the content measured at 70°C is 1,000 Pa·s to 6,000 Pa·s.

## Description

### TECHNICAL FIELD

The present invention relates to a content container.

### BACKGROUND ART

In general, people have a desire to beautify themselves through the application of cosmetics. Accordingly, people feel satisfaction by applying cosmetics to the skin, lips, nails, hair, and the like in various ways.

In the case of lips, cosmetic treatments that make the lips stand out on the face, dissolve lip keratin, and keep the lips smooth and firm are widely used. For such purposes, people apply cosmetics to the lips using lip masks, lipsticks, lip glosses, lip balms, lip tints, and the like.

Lip masks can be manufactured in various forms. For example, a lip mask manufactured in a container type is in use. A lip mask of the container type has a structure in which, when a dial is rotated, a piston rises to discharge a cosmetic product accommodated therein.

Such a lip mask of the container type can be used by bringing the discharge portion into contact with the lips to directly deliver the cosmetic product to the lips, or by transferring the discharged cosmetic product onto the hand or another tool and then delivering it to the lips.

However, a lip mask manufactured in the container type has a relatively complicated structure due to rotation of the dial, ascent of the piston, and the like, and accordingly there is a concern that leakage may occur.

In addition, the concern about leakage of the content may increase depending on the viscosity, hardness, and the like of the formulation in the container-type lip mask. For example, lip masks in an oil formulation, an oil-dispersion formulation, an oil-in-water formulation, a water-in-oil formulation, an oil-wax formulation, and the like are at great risk of leakage of the content. Accordingly, it is necessary to control the leakage of the content by adjusting the viscosity, hardness, and the like of the lip mask. In addition, the need for improvement has also been raised with respect to the operational feel of the dial.

### DISCLOSURE

### TECHNICAL PROBLEM

The present invention has been created to solve the problems of the prior art as described above, and an object of the present invention is to provide a content container that, with respect to a structure in which a pressing part moves up and down as an operating part rotates so that a content is discharged, can sufficiently prevent leakage of the content while improving the feel of use of the operating part, thereby improving user convenience and satisfaction.

The problems of the present invention are not limited to the problems mentioned above, and other problems not mentioned will be clearly understood by those skilled in the art from the description below.

### TECHNICAL SOLUTION

A content container according to one aspect of the present invention comprises: a storage part that accommodates a content; a shaft part that rotates relative to the storage part; and a pressing part that is screw-coupled to the shaft part and pushes up the content by rotation of the shaft part, wherein the content has a viscosity of 1,000 Pa·s to 6,000 Pa·s as measured at 70°C.

Specifically, the content may have a hardness of 85 dyne/cm² to 500 dyne/cm² as measured at 25°C.

Specifically, the content may comprise 5 to 15% by weight of a gelling agent based on the total weight of the content and 0.1 to 5% by weight of a thickener based on the total weight of the content.

Specifically, the gelling agent may comprise a dextrin fatty acid ester.

Specifically, the thickener may comprise one or more selected from the group consisting of silica silylate and a hectorite-based thickener.

Specifically, the content may comprise one or more oily components selected from the group consisting of hydrogenated polyisobutene, pentaerythritol fatty acid esters, phytosterol fatty acid esters, diisostearyl malate, and silicone oil.

Specifically, the content may comprise 50 to 89% by weight of the oily component based on the total weight of the content.

In addition, a content container according to one aspect of the present invention comprises: a storage part that accommodates a content; a discharge part that has a discharge port and is coupled to an upper side of the storage part; an operating part that is rotatably assembled to a lower side of the storage part; and a pressing part that pushes up the content by manipulation of the operating part, wherein the discharge part has a form that engages with both an outer circumference and an inner circumference at the upper end of the storage part, and the operating part has an uneven structure between the operating part and the storage part.

Specifically, the storage part may comprise: an uneven part that is formed by being continuously recessed inward along the height direction in the side wall and limits rotation of the pressing part; and a protruding step that is provided on the outside of the side wall and has a form that interrupts the uneven part in the height direction.

Specifically, the uneven part may have a curved surface shape that is convex inward, and the pressing part may have a groove having a concave curved surface shape on its circumference that engages with the uneven part.

Specifically, the content container may further comprise a cover part having a stopper protrusion that is inserted into the discharge port, wherein the stopper protrusion has a height such that it is partially inserted into the discharge port and is formed in a hollow form.

Specifically, the storage part may further comprise a rim provided so as to protrude on the outside of the side wall, and the discharge part may further comprise a ring-shaped groove that engages with the rim so as to maintain coupling with the storage part.

Specifically, the discharge part surrounds the outer circumference of the upper end of the storage part and is coupled to the storage part so that relative rotation is restricted, and may comprise a sealing rib that protrudes downward and engages with the inner circumference of the upper end of the storage part.

Specifically, the sealing rib may be provided with a sealing protrusion on its outer circumference that abuts against the inner circumference of the upper end of the storage part, and the sealing protrusion may block the gap between the sealing rib and the upper end of the storage part so as to block leakage of the content.

In addition, a content container according to one aspect of the present invention comprises: a storage part that accommodates a content; an operating part that is rotatably assembled to a lower side of the storage part; and a pressing part that pushes up the content by manipulation of the operating part, wherein the operating part comprises a plurality of uneven protrusions provided radially, and the storage part comprises an operational feel generating part that cooperates with the uneven protrusions to provide an operational feel relating to a rotation angle of the operating part and an extent of upward and downward movement of the pressing part.

Specifically, when the operating part rotates by a rotation angle between adjacent uneven protrusions, 0.01 to 0.1 mL of the content may be discharged.

Specifically, the uneven protrusions may be arranged at regular angular intervals along the direction of rotation of the operating part.

Specifically, the content container may comprise a shaft part having a threaded column formed on its side surface and rotating relative to the storage part, wherein the threaded column may have a pitch of 0.1 to 5 mm.

Specifically, the operational feel generating part may comprise: an arm that extends horizontally in a cantilever form from the lower end of the storage part; and a locking protrusion that protrudes downward from the arm.

Specifically, the operating part may further comprise: a center protrusion provided at the center of the inner bottom surface; and a ring-shaped protrusion provided around the center protrusion to guide relative rotation of the storage part with respect to the operating part, wherein the uneven protrusions may be provided around the ring-shaped protrusion.

### ADVANTAGEOUS EFFECTS OF INVENTION

The content container of the present invention has the following effects.

The content container according to the present invention has a structure in which the pressing part rises as the operating part rotates relative to the storage part so that the content is discharged, while facilitating the assembly of a plurality of components and achieving sufficient sealing to prevent the content from leaking to the outside through gaps formed between components, such as between the operating part and the storage part and between the storage part and the discharge part.

In addition, the content container according to the present invention can secure rotational stability of the shaft part when the shaft part rotates integrally with the operating part so as to improve the feel of use, and can generate a sound when the operating part rotates so as to secure user convenience.

The effects of the present invention are not limited to the effects mentioned above, and other effects not mentioned will be clearly understood by those skilled in the art from the description in the claims.

### BRIEF DESCRIPTION OF DRAWINGS

FIG. 1 is a perspective view of a content container according to an embodiment of the present invention.
FIG. 2 is an exploded perspective view of the content container according to an embodiment of the present invention.
FIG. 3 is an exploded perspective view of the content container according to an embodiment of the present invention.
FIG. 4 is a cross-sectional view of the content container according to an embodiment of the present invention.
FIG. 5 is a perspective view of a cover part of the content container according to an embodiment of the present invention.
FIG. 6 is a side view of a storage part of the content container according to an embodiment of the present invention.
FIG. 7 is a perspective view of the storage part of the content container according to an embodiment of the present invention.
FIG. 8 is a perspective view of the storage part of the content container according to an embodiment of the present invention.
FIG. 9 is a perspective view of an auxiliary coupling part of the content container according to an embodiment of the present invention.
FIG. 10 is a perspective view of a discharge part of the content container according to an embodiment of the present invention.
FIG. 11 is a perspective view of an operating part of the content container according to an embodiment of the present invention.
FIG. 12 is a perspective view showing the coupling of the auxiliary coupling part and the cover part in the content container according to an embodiment of the present invention.
FIG. 13 is a perspective view showing the coupling of the auxiliary coupling part and the operating part in the content container according to an embodiment of the present invention.
FIG. 14 is a perspective view showing the coupling of the operating part and the shaft part in the content container according to an embodiment of the present invention.
FIG. 15 is an enlarged view of a portion where the uneven part and the groove engage each other in the content container according to an embodiment of the present invention.
FIG. 16 is an enlarged view of portion 'A' in FIG. 4.
FIG. 17 is an enlarged view of portion 'B' in FIG. 4.
FIG. 18 is an enlarged view of portion 'C' in FIG. 4.
FIG. 19 is an enlarged view of portion 'D' in FIG. 4.

### DESCRIPTION OF EMBODIMENTS

The objects, specific advantages, and novel features of the present invention will become more apparent from the following detailed description and preferred embodiments in conjunction with the accompanying drawings. It should be noted that, in adding reference numerals to constituent elements in each drawing of this specification, the same constituent elements have been assigned the same numbers as much as possible even if they are shown in different drawings. In addition, in describing the present invention, if it is determined that detailed descriptions of related known technologies may unnecessarily obscure the gist of the present invention, the detailed descriptions thereof will be omitted.

When a part is said to "include" a component, this does not mean that other components are excluded, but rather that it may further comprise other components, unless specifically stated to the contrary.

Hereinafter, preferred embodiments of the present invention will be described in detail with reference to the accompanying drawings. For reference, the present invention includes a content container as described below and a cosmetic product comprising a content accommodated in the content container. In this case, the content may be in solid or fluid form, without limitation, and in the case of a fluid, viscosity, formulation, and the like may be variously determined. In addition, the content may be a cosmetic substance, a medical substance, or an edible substance that is applied to a body part such as the lips, or a substance that is administered into the body, and may have an unlimited range of applications.

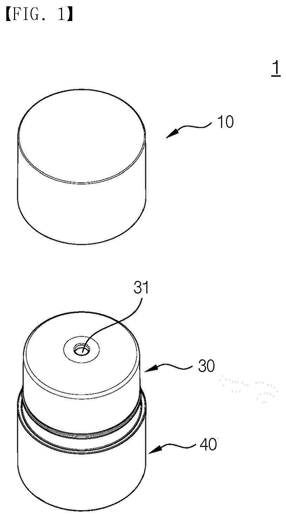

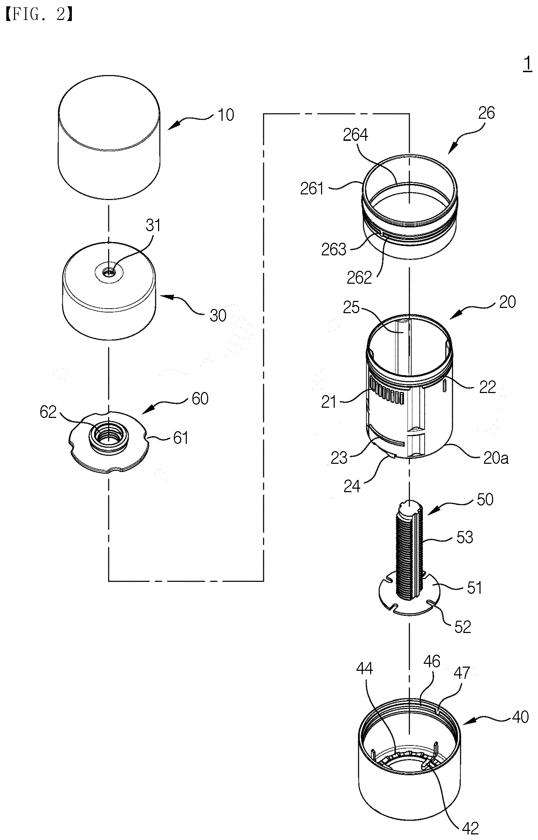

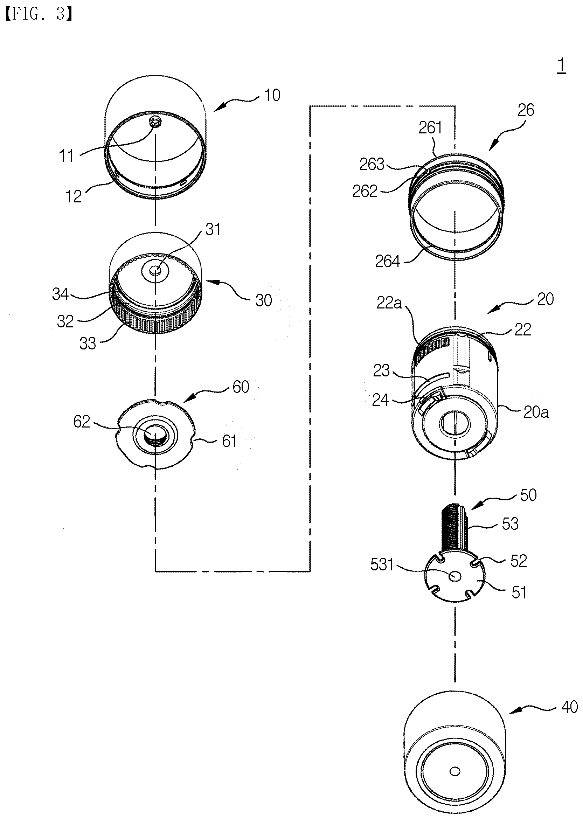

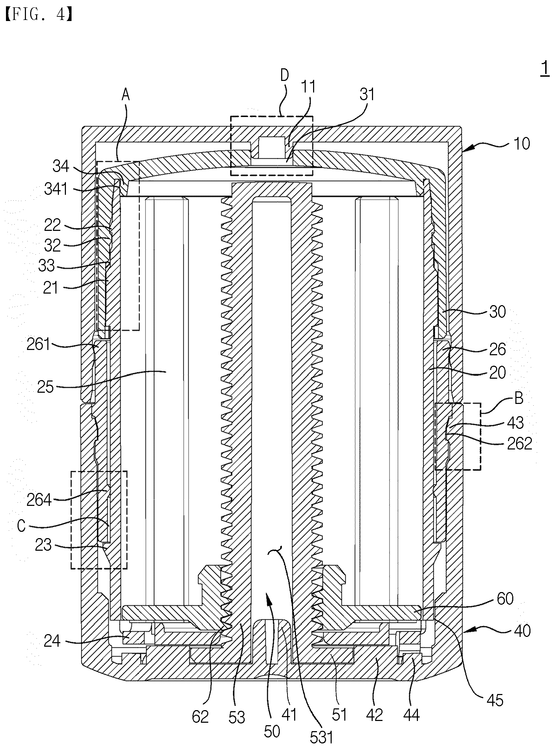

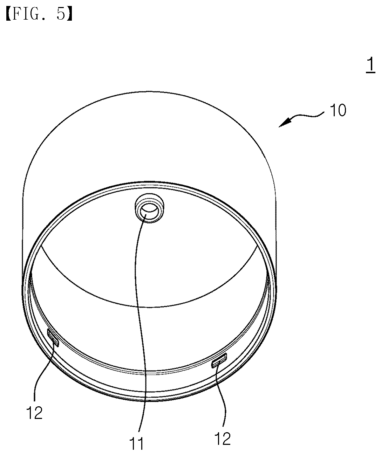

FIG. 1 is a perspective view of a content container according to an embodiment of the present invention, FIGS. 2 and 3 are exploded perspective views of the content container according to an embodiment of the present invention, FIG. 4 is a cross-sectional view of the content container according to an embodiment of the present invention, and FIG. 5 is a perspective view of a cover part of the content container according to an embodiment of the present invention.

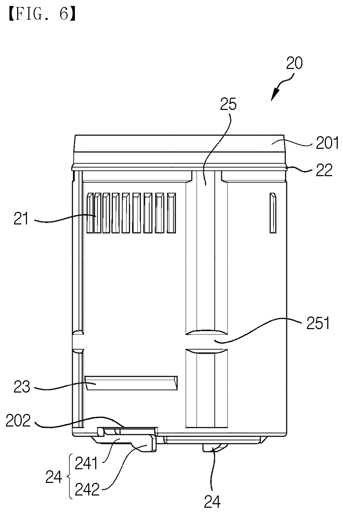

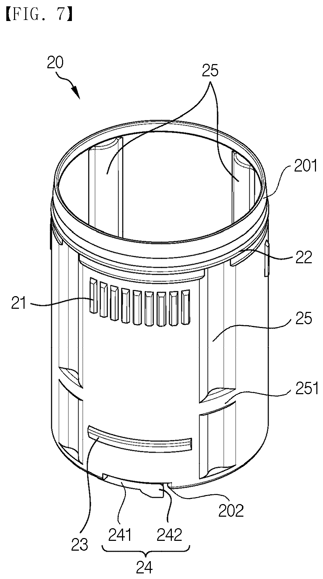

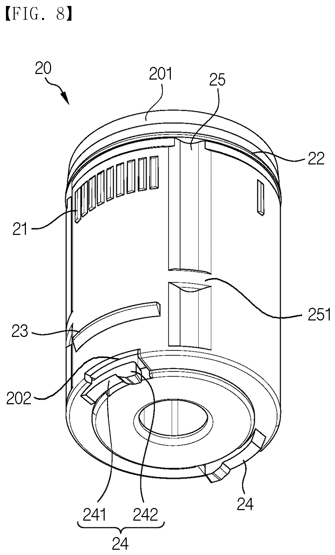

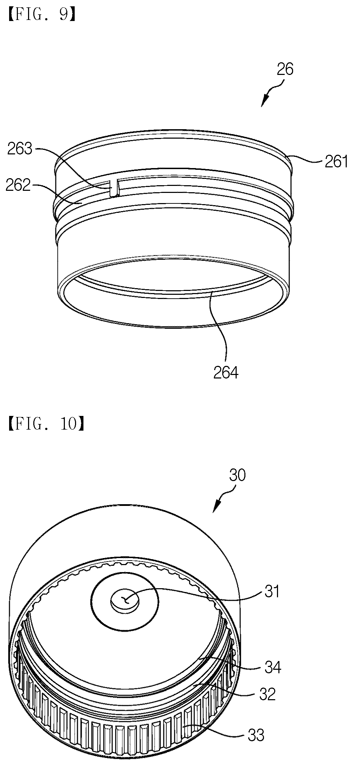

FIG. 6 is a side view of a storage part of the content container according to an embodiment of the present invention, FIGS. 7 and 8 are perspective views of the storage part of the content container according to an embodiment of the present invention, and FIG. 9 is a perspective view of an auxiliary coupling part of the content container according to an embodiment of the present invention.

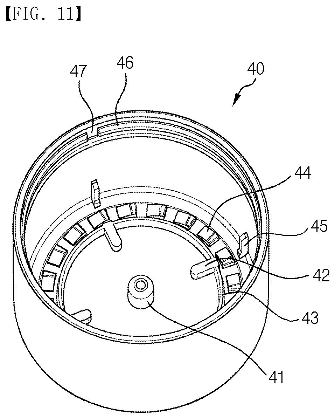

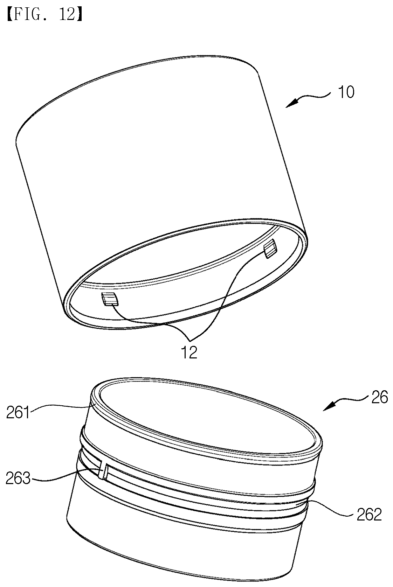

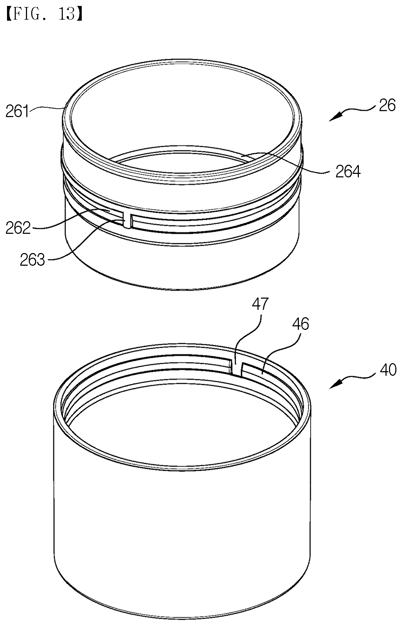

FIG. 10 is a perspective view of a discharge part of the content container according to an embodiment of the present invention, FIG. 11 is a perspective view of an operating part of the content container according to an embodiment of the present invention, FIG. 12 is a perspective view showing the coupling of the auxiliary coupling part and the cover part in the content container according to an embodiment of the present invention, and FIG. 13 is a perspective view showing the coupling of the auxiliary coupling part and the operating part in the content container according to an embodiment of the present invention.

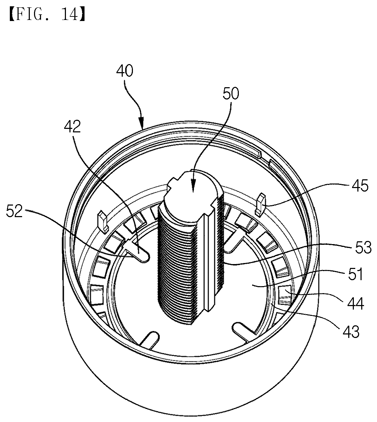

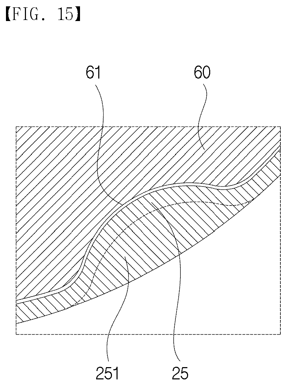

FIG. 14 is a perspective view showing the coupling of the operating part and the shaft part in the content container according to an embodiment of the present invention, and FIG. 15 is an enlarged view of a portion where the uneven part and the groove engage each other in the content container according to an embodiment of the present invention.

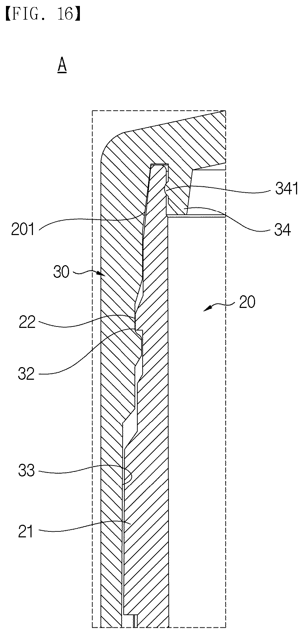

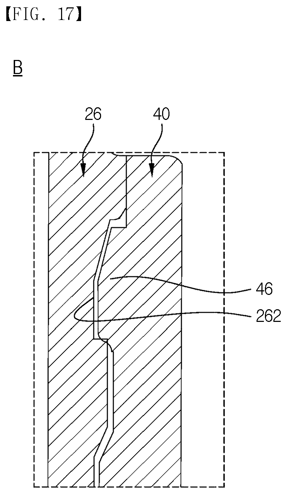

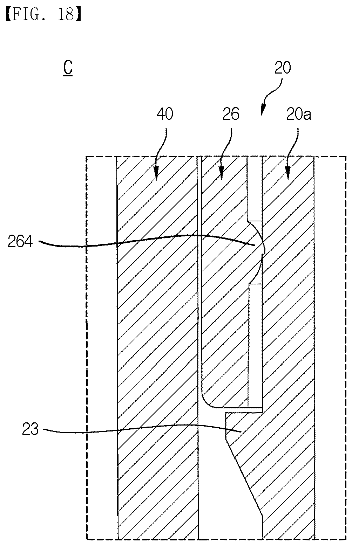

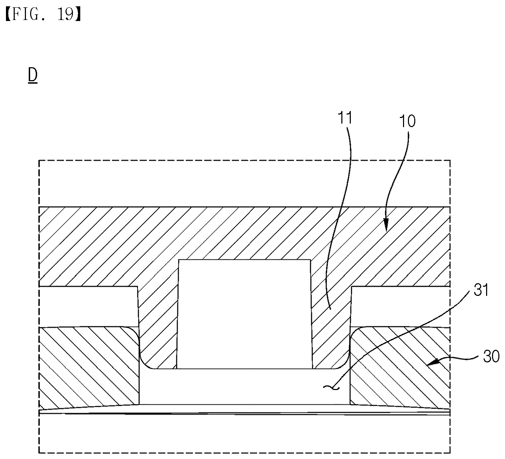

FIG. 16 is an enlarged view of portion 'A' in FIG. 4, FIG. 17 is an enlarged view of portion 'B' in FIG. 4, FIG. 18 is an enlarged view of portion 'C' in FIG. 4, and FIG. 19 is an enlarged view of portion 'D' in FIG. 4.

Referring to FIGS. 1 to 19, a content container 1 according to an embodiment of the present invention consists of a plurality of components, is manufactured by assembling the plurality of components, and is provided with an assembly structure, a sealing structure, and the like that fundamentally prevent the content from leaking to the outside.

The content container 1 according to an embodiment of the present invention comprises a cover part 10, a storage part 20, a discharge part 30, an operating part 40, a shaft part 50, and a pressing part 60. Hereinafter, a case in which the content container 1 of the present embodiment is cylindrical will be described, but it goes without saying that it can be implemented in various shapes such as a polygon.

The cover part 10 can be coupled above the discharge port 31 to close off the upper portion of the storage part 20. A stopper protrusion 11 may be provided inside the cover part 10. The stopper protrusion 11 is fitted into the discharge port 31 provided at the upper portion of the discharge part 30 so as to close off the storage part 20 that accommodates the content.

The cover part 10 is provided in the form of a tube having an open lower portion and a closed upper portion, and a stopper protrusion 11 that seals the discharge port 31 of the discharge part 30 may be provided on the lower surface of the upper portion so as to protrude downward. In this case, the stopper protrusion 11 may be partially inserted into the discharge port 31 as shown in FIG. 19 and the like. That is, the stopper protrusion 11 may have a relatively small height compared to the depth of the discharge port 31.

In addition, the stopper protrusion 11 may be formed in a hollow form. The outer circumference of the stopper protrusion 11 engages with the inner circumference of the discharge port 31 so as to prevent leakage of the content, and for this purpose, the stopper protrusion 11 has a size that fits completely into the discharge port 31. In this case, if the stopper protrusion 11 has a solid-filled form, the stopper protrusion 11 increases the internal pressure of the storage part 20 when the cover part 10 is coupled to the discharge part 30.

In this state, if the user subsequently removes the cover part 10, there is a concern that the content may seep out due to residual pressure. Accordingly, the stopper protrusion 11 of the present embodiment has a height smaller than the depth of the discharge port 31 so as to minimize the rise in internal pressure of the storage part 20. In addition, since the stopper protrusion 11 has a hollow form, even when the stopper protrusion 11 is inserted into the discharge port 31, the rise in internal pressure of the storage part 20 can be resolved by utilizing the inner space of the stopper protrusion 11.

A snap protrusion 12 may be provided on the inner circumferential surface of the cover part 10. The snap protrusion 12 may be used to fasten the cover part 10 to an auxiliary coupling part 26 described later. The snap protrusion 12 may be provided in plurality and may be assembled with an outer protrusion 261 provided on the upper rim of the auxiliary coupling part 26. The plurality of snap protrusions 12 may be arranged radially, and the outer protrusion 261 may be provided in a ring form so as not to limit the direction in which the cover part 10 is fastened to the auxiliary coupling part 26.

Of course, conversely, it is also possible for a snap protrusion to be provided on the auxiliary coupling part 26 and an inner protrusion that engages with the snap protrusion 12 to be formed on the cover part 10. Various other fastening structures may also be applied.

The storage part 20 accommodates a content. The storage part 20 may have a shape in which the upper portion is open and a hole (not shown) into which the shaft part 50 is inserted is formed at the lower portion. The discharge part 30 and the cover part 10 may be coupled to the upper portion of the storage part 20.

The discharge part 30 and the storage part 20 may be coupled so that relative rotation is restricted. That is, the discharge part 30 and the storage part 20 are coupled so that mutual rotation is not possible and can rotate integrally. On the other hand, the operating part 40 is provided so as to be capable of rotating relative to the storage part 20. Accordingly, when the user holds the discharge part 30 and rotates the operating part 40, relative rotation of the operating part 40 with respect to the storage part 20 can be achieved. Of course, the user can also hold the operating part 40 and rotate the discharge part 30, at which time relative rotation of the discharge part 30 can be achieved.

The storage part 20 may comprise a serration protrusion 21, a rim 22, a support protrusion 23, an operational feel generating part 24, an uneven part 25, and an auxiliary coupling part 26. Each configuration will be described in detail below.

The storage part 20 may be provided with a rim 22 so as to protrude on the outside of the side wall. The rim 22 may have a ring form. The storage part 20 and the discharge part 30 may be fastened together by engagement of the rim 22 of the storage part 20 and the ring-shaped groove 32 of the discharge part 30.

When the discharge part 30 is coupled to one side of the storage part 20, it may be provided with a ring-shaped groove 32 at a position corresponding to the rim 22. As shown in FIG. 16, the ring-shaped groove 32 may engage with the rim 22 so that the discharge part 30 maintains coupling with the storage part 20.

In addition, the storage part 20 and the discharge part 30 may be fastened by engagement of the serration protrusion 21 of the storage part 20 and the fixing protrusion 33 of the discharge part 30. The fastening between the rim 22 and the ring-shaped groove 32 is for preventing the discharge part 30 from being separated from the storage part 20 and does not restrict relative rotation of the discharge part 30 with respect to the storage part 20. However, the fastening between the serration protrusion 21 and the fixing protrusion 33 may be provided to restrict relative rotation of the discharge part 30 with respect to the storage part 20.

The serration protrusion 21 may be provided along the circumferential direction on the outside of the side wall of the storage part 20. The serration protrusion 21 may have a form in which vertical unit teeth of a preset height are arranged at regular intervals along the circumference of the side wall of the storage part 20. The unit teeth may be arranged at intervals smaller than the size of each unit tooth or at intervals similar to the size of the unit tooth, and the like, and the fixing protrusion 33 may be formed on the discharge part 30 corresponding to the intervals between the unit teeth.

In addition, 5 to 10 (preferably 8 or 9) unit teeth may be arranged at regular intervals between two adjacent uneven parts 25. In this case, the serration protrusion 21 is spaced apart from the uneven part 25 by a first interval in the circumferential direction of the storage part 20, and the first interval may be set larger than the regular interval at which the unit teeth are arranged.

The reason that 5 or more unit teeth are arranged to form the serration protrusion 21 is that structural rigidity can be supplemented and improved compared to a case where the serration protrusions 21 are arranged at spaced intervals. That is, in the present embodiment, by forming the intervals between unit teeth small in the serration protrusion 21, the coupling force between the storage part 20 and the discharge part 30 can be sufficiently secured, and stable integral rotation of the discharge part 30 and the storage part 20 can be guaranteed.

The serration protrusion 21 may be provided below the rim 22 on the outside of the side wall of the storage part 20. In this case, when the discharge part 30 is coupled to the storage part 20, the lower end of the discharge part 30 passes through the rim 22 and then passes through the serration protrusion 21.

In order to prevent the fixing protrusion 33 that engages with the serration protrusion 21 in the discharge part 30 from catching on the rim 22 of the storage part 20, the discharge part 30 may be provided such that the inner diameter of the lower portion where the fixing protrusion 33 is provided is relatively larger than the inner diameter of the upper portion where the ring-shaped groove 32 is provided.

Accordingly, when the discharge part 30 is coupled to the storage part 20, the fixing protrusion 33 provided at the lower portion of the discharge part 30 can pass by without interfering with the rim 22 of the storage part 20 and then engage with the serration protrusion 21 of the storage part 20.

The plurality of fixing protrusions 33 provided on the discharge part 30 may be arranged along the inner circumferential surface of the discharge part 30. That is, the fixing protrusion 33 of the discharge part 30 may be provided in the form of teeth corresponding to the serration protrusion 21 of the storage part 20.

The storage part 20 may further comprise a support protrusion 23. The support protrusion 23 may be used for seating the auxiliary coupling part 26, as shown in FIG. 18. For reference, the auxiliary coupling part 26 may also be interpreted as forming a part of the storage part 20, and the portion of the storage part 20 excluding the auxiliary coupling part 26 may be referred to as the storage body 20a for convenience.

The support protrusion 23 may be provided in the form of a bar that is horizontal on the outer circumferential surface at the lower portion of the storage body 20a. The support protrusion 23 may be provided in plurality and can support the lower edge of the auxiliary coupling part 26. That is, the support protrusion 23 can restrict the auxiliary coupling part 26 from moving downward relative to the storage body 20a.

An operational feel generating part 24 may be provided at the lower portion of the storage part 20. The operational feel generating part 24 is provided at the lower end of the storage part 20 and can cooperate with the uneven protrusions 44 provided on the operating part 40 during relative rotation of the operating part 40 to provide an operational feel relating to the rotation angle and the extent of upward and downward movement of the pressing part 60. The operational feel generating part 24 can cooperate with the uneven protrusions 44 to provide stimuli such as auditory stimulation and tactile stimulation to the user. For example, the operational feel generating part 24 and the uneven protrusions 44 can generate an operational feel as the operational feel generating part 24 passes over the uneven protrusions 44 when the operating part 40 is rotated, or can generate a tactile feel as the operational feel generating part 24 catches on the uneven protrusions 44. Accordingly, the user can feel, based on a clicking sound or click sensation, the degree of rotation of the operating part 40, the extent of upward and downward movement of the pressing part 60, and the like, and can experience an operational feel during use. In addition, due to the engagement of the operational feel generating part 24 and the uneven protrusions 44, the user can uniformly adjust the rotation angle of the operating part 40, can adjust the extent of upward and downward movement of the pressing part 60, and can control the discharge amount of the content.

At least one operational feel generating part 24 may be provided along the lower edge of the storage part 20. The operational feel generating part 24 of the storage part 20 may be provided in plurality radially, and may be formed to include an arm 241 and a locking protrusion 242 and the like. The arm 241 may extend in a cantilever form from the lower end of the storage part 20. For example, the arm 241 may extend in a direction that can have an elastic function (for example, a direction including a horizontal component). That is, the arm 241 may be connected in an L-shape at the lower end of the storage part 20.

The arm 241 may be elastic. That is, the arm 241 can be bent by an external force and, when the external force disappears, return to a straight form by elastic force. The arm 241 has one end fixed to the lower end of the storage part 20 and when an external force is applied to the locking protrusion 242 formed at the other end, the arm 241 deforms by bending upward, and when the external force applied to the locking protrusion 242 disappears, the arm 241 can return to its original form.

The storage part 20 may have a recessed end 202 that is recessed upward at the portion of the lower end portion where the operational feel generating part 24 is located and accommodates at least a part of the arm 241. The bottom portion of the lower end of the storage part 20 may be partially cut out and penetrated so that the recessed end 202 can be formed. However, even if the bottom of the storage part 20 is penetrated to provide the recessed end 202, since the circumference of the pressing part 60 and the inner wall of the storage part 20 have a mutually sealed structure, the content stored above the pressing part 60 may not leak through the recessed end 202.

The recessed end 202 is provided to accommodate the free end portion of the arm 241 where the locking protrusion 242 is provided, and can form a clearance space in which the arm 241 deforms upward. When the storage part 20 rotates and the operational feel generating part 24 passes over the uneven protrusions 44, the arm 241 of the operational feel generating part 24 can deform upward toward the inside of the recessed end 202.

The locking protrusion 242 may protrude downward from the arm 241. The locking protrusion 242 protrudes downward from the end of the arm 241 and may be provided in a form including an inclined surface and a vertical surface and the like. The locking protrusion 242 may have a curved inclined surface along the direction of rotation of the operational feel generating part 24, and a vertical surface formed at the point where the inclined surface ends. The inclined surface and vertical surface of the locking protrusion 242 can cooperate with the plurality of uneven protrusions 44 provided on the operating part 40 to generate an operational feel.

The storage part 20 is provided with an uneven part 25. The uneven part 25 is formed at regular intervals on the inner circumferential surface of the storage part 20, and suppresses rotation of the pressing part 60 provided inside the storage part 20, thereby enabling the pressing part 60 to rise or descend.

The uneven part 25 may be formed by being continuously recessed inward along the height direction in the side wall of the storage part 20. That is, the uneven part 25 may have a form in which the side wall is recessed inward, so that it protrudes on the inner surface of the side wall and is recessed on the outer surface of the side wall.

The uneven part 25 may have a curved surface shape that is convex inward. In this case, the pressing part 60 may have a groove 61 having a concave curved surface shape on its circumference that engages with the uneven part 25. The storage part 20 needs to engage with the pressing part 60 in an uneven structure in order to suppress rotation of the pressing part 60, and in the present embodiment, by making the uneven structure into a curved surface form, leakage of the content between the storage part 20 and the pressing part 60 can be minimized.

The convex curved surface of the uneven part 25 may be formed in an arc shape having an angle within 180 degrees (preferably within 150 degrees) as shown in FIG. 15. That is, the curved surface of the uneven part 25 may have a relatively gently convex form in the shape of an arc smaller than a semicircle, and both ends of the curved surface of the uneven part 25 may be connected to the side wall of the storage part 20 at an angle of 120 degrees or more. For reference, FIG. 15 shows the convex curved surface of the uneven part 25 and the groove 61 of the pressing part 60 as if they were separated for ease of understanding, but in reality, they are in close contact so that the content does not leak.

The height of the uneven part 25 can determine the range of elevation of the pressing part 60. The upper end of the uneven part 25 can determine the point at which the pressing part 60 rises to a maximum. The uneven part 25 may have a height such that the upper end is located above the rim 22. However, since the uneven part 25 has a form that is recessed on the outer surface of the side wall of the storage part 20, the upper end of the uneven part 25 may be filled by the rim 22 so that the content does not leak along the uneven part 25. That is, the rim 22 may be provided in a form that fills the upper portion of the uneven part 25 on the outside of the side wall.

In addition, in a similar context, the uneven part 25 may be provided with a protruding step 251. The protruding step 251 is provided on the outside of the side wall and may have a form that interrupts the uneven part 25 in the height direction. Even if the content leaks into the lower portion of the recessed portion on the outer surface of the side wall due to the uneven part 25, the content may be blocked by the protruding step 251 and leakage upward may be blocked.

For example, when the content leaks between the storage part 20 and the operating part 40, the content can flow into the lower portion of the recessed curved surface of the uneven part 25 provided on the side surface of the storage part 20. Specifically, when the shaft part 50 rotates, the content undergoes a phase change due to the rotational force of the threaded column 52, and content with increased flowability can flow out along the gap between the threaded column 52 and the pressing part 60. At this time, the leaked content may creep upward along the outer circumferential surface of the storage part 20, which may result in leakage.

At this time, if the content leaks upward along the recessed curved surface of the uneven part 25, there is a problem that it leaks to the outside through the gap between the discharge part 30 and the storage part 20. However, according to the present embodiment, the protruding step 251 may be filled in at least one or more positions in the vertical direction among the concave curved surface portions of the uneven part 25. The protruding step 251 can interrupt the concave curved surface of the uneven part 25, thereby blocking leakage of the content through the recessed curved surface of the uneven part 25.

The protruding step 251 may be provided with its outer surface continuous with the side surface of the storage part 20, but it is also possible for it to be provided to protrude further than the side surface of the storage part 20, similar to the rim 22.

As the uneven part 25 has a sufficient height in the side wall of the storage part 20 to secure the range of elevation of the pressing part 60, the serration protrusion 21 of the storage part 20 may be arranged at the same position as at least a portion of the uneven part 25 in the height direction. In this case, the serration protrusion 21 may be provided between the plurality of uneven parts 25.

The auxiliary coupling part 26 is provided on the outside of the storage body 20a and may be provided so as to be rotatable relative to the storage body 20a. The auxiliary coupling part 26 may comprise an outer protrusion 261, a first coupling groove 262, a first coupling protrusion 263, and an inner protrusion 264.

The outer protrusion 261 may be provided on the upper rim of the auxiliary coupling part 26. The outer protrusion 261 may be assembled with the plurality of snap protrusions 12 provided on the cover part 10. However, even if the outer protrusion 261 of the auxiliary coupling part 26 and the snap protrusion 12 of the cover part 10 are coupled, relative rotation of the cover part 10 with respect to the auxiliary coupling part 26 may be permitted.

The first coupling groove 262 may be provided so as to be recessed in a ring form on the outer circumferential surface of the auxiliary coupling part 26. As shown in FIG. 17, the first coupling groove 262 may be assembled with the second coupling protrusion 46 of the operating part 40. At least the first coupling groove 262 among the first coupling groove 262 and the second coupling protrusion 46 may be provided in a ring form.

The first coupling protrusion 263 may be provided so as to protrude on the outer circumferential surface of the auxiliary coupling part 26, and at least a part thereof may be arranged overlapping the first coupling groove 262. The first coupling protrusion 263 may be provided in plurality and may be assembled with the plurality of second coupling grooves 47 provided on the operating part 40.

In the operating part 40, the second coupling groove 47 may be provided in a form that interrupts the second coupling protrusion 46, and the angle at which the first coupling protrusion 263 engages with the second coupling groove 47 may be limited. That is, when the auxiliary coupling part 26 and the operating part 40 are fastened to each other via the first coupling protrusion 263 and the second coupling groove 47, relative rotation of the operating part 40 with respect to the auxiliary coupling part 26 is restricted. Accordingly, when the user rotates the operating part 40, the auxiliary coupling part 26 also rotates together. However, as described above, the cover part 10 can rotate relative to the auxiliary coupling part 26.

The inner protrusion 264 may be provided so as to protrude on the inner circumferential surface of the auxiliary coupling part 26 so as to be in close contact with the outer circumferential surface of the storage body 20a. The inner protrusion 264 is provided in a ring form and may be used to achieve sealing between the auxiliary coupling part 26 and the storage body 20a.

As shown in FIG. 18, when the inner protrusion 264 of the auxiliary coupling part 26 is in close contact with the outer circumferential surface of the storage body 20a, leakage of the content to the outside through the space between the inner circumferential surface of the auxiliary coupling part 26 and the outer circumferential surface of the storage body 20a can be prevented.

In particular, the storage body 20a has an uneven part 25 on its side wall, and a protruding step 251 is provided at one point in the height direction in the recessed curved surface portion of the uneven part 25. The inner protrusion 264 of the auxiliary coupling part 26 is provided at the same height as the protruding step 251 and can be in close contact with the protruding step 251. Through this, the protruding step 251 can effectively prevent leakage between the storage body 20a and the auxiliary coupling part 26.

With respect to the auxiliary coupling part 26, the cover part 10 may be coupled above and the operating part 40 may be coupled below. In this case, the auxiliary coupling part 26 and the cover part 10 are fastened in a relatively rotatable manner as the outer protrusion 261 of the auxiliary coupling part 26 and the snap protrusion 12 of the cover part 10 engage with each other, whereas the auxiliary coupling part 26 and the operating part 40 may be fastened so that relative rotation therebetween is not possible as the first coupling protrusion 263 of the auxiliary coupling part 26 and the second coupling groove 47 of the operating part 40 engage with each other. In addition, the auxiliary coupling part 26 is provided so that relative rotation with respect to the storage body 20a is not restricted.

When the user holds the cover part 10 tightly so that its sides are compressed and rotates the operating part 40, there is a risk that the content may be discharged if the auxiliary coupling part 26 rotates relative to the operating part 40. However, in the present embodiment, by configuring the operating part 40 and the auxiliary coupling part 26 to rotate integrally, when the user presses the sides of the cover part 10 or the like to a degree sufficient to suppress rotation of the auxiliary coupling part 26, rotation of the operating part 40, which is integrally coupled with the auxiliary coupling part 26, is suppressed, thereby eliminating the risk of the content being discharged.

The present embodiment can define the operating part 40, the shaft part 50, and the auxiliary coupling part 26, which are coupled to rotate integrally, as a first group, and can also define the discharge part 30, the storage body 20a, and the pressing part 60, which are coupled to rotate integrally, as a second group. The first group and the second group can rotate relative to each other, and in the present embodiment, a case where the first group is stationary and the second group rotates, or a case where the second group is stationary and the first group rotates, and the like are possible.

For example, a case where the first group is stationary and the second group rotates may be a state where the pressing part 60 and the like rotate as the user holds the operating part 40 and turns the discharge part 30. Conversely, a case where the second group is stationary and the first group rotates may be a state where the shaft part 50 and the like rotate as the user holds the discharge part 30 and turns the operating part 40.

The discharge part 30 may be provided in a form in which the lower portion is open and the upper portion covers the open upper portion of the storage part 20. The discharge part 30 may be provided with a discharge port 31 for discharging the content, and may be assembled so as to be fixed to the outer circumferential surface of the upper portion of the storage part 20.

The discharge part 30 may surround the outer circumference of the upper end of the storage part 20 and be coupled to the storage part 20 so that relative rotation is restricted. The discharge part 30 comprises a discharge port 31, a ring-shaped groove 32, a fixing protrusion 33, and a sealing rib 34. The discharge port 31 may be provided at the upper portion to allow the content accommodated in the storage part 20 to be discharged to the outside. The discharge port 31 can be closed off when the stopper protrusion 11 of the cover part 10 is inserted.

The ring-shaped groove 32 may be provided in a ring form on the inner circumferential surface of the discharge part 30 and may be assembled with the rim 22 provided in a ring form on the outer circumferential surface of the storage part 20. Of course, it is also possible for the ring-shaped groove 32 to be provided on the outer circumferential surface of the storage part 20 and the rim 22 to be provided on the inner circumferential surface of the discharge part 30.

The ring-shaped groove 32 of the discharge part 30 and the rim 22 of the storage part 20 are used to maintain a mutually fastened state and do not restrict relative rotation. However, relative rotation of the discharge part 30 with respect to the storage part 20 is restricted, and this is achieved by the fixing protrusion 33 described later.

The fixing protrusion 33 engages with the serration protrusion 21 of the storage part 20 so as to restrict relative rotation of the discharge part 30 with respect to the storage part 20. The serration protrusion 21 of the storage part 20 has a form in which a plurality of unit teeth are arranged at regular intervals, and the fixing protrusion 33 can be inserted into the gap between adjacent unit teeth. Accordingly, relative rotation of the discharge part 30 with respect to the storage part 20 is suppressed as the fixing protrusion 33 of the discharge part 30 engages with the serration protrusion 21 of the storage part 20.

In addition, the discharge part 30 may be provided with a plurality of fixing protrusions 33, and furthermore, the fixing protrusions 33 may be arranged in the form of teeth. As shown in the drawings, the fixing protrusion 33 may be arranged at regular intervals over the entire inner circumferential surface of the discharge part 30. In this case, the intervals between the fixing protrusions 33 may correspond to the size of the unit tooth.

As shown in FIG. 16, the discharge part 30 may be provided such that the lower portion where the fixing protrusion 33 is provided has a relatively larger inner diameter than the upper portion where the ring-shaped groove 32 is provided. This is to prevent the fixing protrusion 33 of the discharge part 30 from interfering with the rim 22 of the storage part 20 with which the ring-shaped groove 32 of the discharge part 30 engages.

The sealing rib 34 protrudes downward from the lower surface of the upper wall of the discharge part 30 and may be provided so as to engage with the inner circumference of the upper end of the storage part 20. Since the outer circumference of the upper end of the storage part 20 is covered by the side wall of the discharge part 30 and the inner circumference abuts against the sealing rib 34, double sealing between the storage part 20 and the discharge part 30 can be achieved.

The sealing rib 34 may be provided in a ring form so as to be in close contact with the entire inner circumference of the upper end of the storage part 20. In addition, the sealing rib 34 may be provided with a sealing protrusion 341 on its outer circumference that abuts against the inner circumference of the upper end of the storage part 20. The sealing protrusion 341 can block the gap between the sealing rib 34 and the upper end of the storage part 20 so as to block leakage of the content.

In order to reinforce the sealing coupling between the discharge part 30 and the storage part 20, as shown in FIG. 16, a thickness-reduced portion 201 may be provided at the upper end of the side wall of the storage part 20. The thickness-reduced portion 201 is provided at the very top of the side wall of the storage part 20, and the rim 22 provided on the side wall of the storage part 20 may be provided below the thickness-reduced portion 201.

The thickness-reduced portion 201 is provided at the very top of the side wall of the storage part 20 and may have a form in which the thickness in the inner-outer direction at the upper side is relatively smaller than at the lower side. For example, the thickness-reduced portion 201 of the storage part 20 may have a form in which the thickness gradually decreases as it goes upward, and the inner surface of the discharge part 30 may have a corresponding form. That is, the portion of the side wall of the discharge part 30 facing the thickness-reduced portion 201 of the storage part 20 may have a form in which the thickness in the inner-outer direction gradually increases as it goes upward.

The operating part 40 may have a form in which the upper portion is open and the lower portion is closed, and may be provided to cover the lower side of the storage part 20. In addition, the operating part 40 is rotatably assembled to the lower side of the storage part 20. Since the pressing part 60 rises or descends by the rotational manipulation of the operating part 40, the operating part 40 can control the discharge of the content.

The operating part 40 comprises a center protrusion 41, a peripheral protrusion 42, and a ring-shaped protrusion 43 for assembly of the shaft part 50, and may comprise the ring-shaped protrusion 43, uneven protrusions 44, and a seating protrusion 45 for seating of the storage part 20. In addition, the operating part 40 may comprise a second coupling protrusion 46 and a second coupling groove 47 for fastening with the auxiliary coupling part 26.

The center protrusion 41 is provided at the center of the inner bottom surface of the operating part 40 and can engage with the shaft part 50. The center protrusion 41 protrudes upward from the bottom surface of the operating part 40, and the center protrusion 41 of the operating part 40 may be inserted into the insertion port 521 of the shaft part 50. Through this, the centers of the operating part 40 and the shaft part 50 can be aligned.

The peripheral protrusion 42 is provided around the center protrusion 41. The peripheral protrusion 42 may be provided in plurality radially around the center protrusion 41. The peripheral protrusion 42 can engage with an insertion groove 511 provided on the shaft part 50. A plurality of insertion grooves 511 are also provided on the shaft part 50 corresponding to the plurality of peripheral protrusions 42 provided on the operating part 40, and when the peripheral protrusion 42 and the insertion groove 511 engage with each other, relative rotation of the shaft part 50 with respect to the operating part 40 can be restricted.

The ring-shaped protrusion 43 is provided around the center protrusion 41 and may be provided to connect the plurality of peripheral protrusions 42. The ring-shaped protrusion 43 is provided in a form connecting the outer sides of the peripheral protrusions 42 and allows the fixing plate 51 of the shaft part 50 to be seated inside. Through this, the ring-shaped protrusion 43 can ensure that the shaft part 50 rotates stably without wobbling.

In addition, the ring-shaped protrusion 43 can stably guide the relative rotation of the storage part 20 with respect to the operating part 40. Specifically, at least a portion of the operational feel generating part 24, namely the locking protrusion 242, provided on the storage part 20, can face the outer side of the ring-shaped protrusion 43 in the inner-outer direction. Accordingly, since the storage part 20 rotates while being guided along the outer circumferential surface of the ring-shaped protrusion 43, the ring-shaped protrusion 43 can suppress positional deviation and the like during rotation of the storage part 20.

That is, the ring-shaped protrusion 43 can allow the centers of the shaft part 50 and the operating part 40 to be aligned through the inner end, and can allow the centers of the storage part 20 and the operating part 40 to be aligned through the outer end. Through this, the centers of the storage part 20, the operating part 40, and the shaft part 50 can be mutually aligned to secure coaxiality.

The uneven protrusions 44 may be provided around the peripheral protrusion 42. In particular, the uneven protrusions 44 are provided around the ring-shaped protrusion 43, and cooperate with the operational feel generating part 24 provided on the storage part 20 to provide an operational feel when the operating part 40 and the storage part 20 rotate relative to each other. The operational feel may be a sound or click sensation generated as the operational feel generating part 24 and the uneven protrusions 44 interact. The click sensation may be a sensation that the user can feel through the operating part 40, the storage part 20, or the like. In detail, the click sensation may be a sensation of vibration transmitted to the user or a sensation of decreasing resistance during rotation of the operating part 40.

The uneven protrusions 44 may be provided in plurality radially around the ring-shaped protrusion 43. In order to provide a continuous operational feel when the operating part 40 is rotated, the plurality of uneven protrusions 44 have a relatively greater number than the peripheral protrusions 42 and may be arranged in a tooth shape.

In addition, the uneven protrusions 44 may be regularly arranged around the ring-shaped protrusion 43. That is, the uneven protrusions 44 may be arranged spaced apart at equal intervals or equal angles.

In this case, when the operating part 40 rotates by the angle between adjacent uneven protrusions 44, the distance that the pressing part 60 moves up and down is constant, and the pitch of the threaded column 52 is also constant, so accordingly, the amount of content discharged by the operating part 40 can be constant.

The content container according to an embodiment of the present invention can control the discharge amount of the content by adjusting the pitch of the threaded column 52 and the arrangement angle of the uneven protrusions 44. The content container according to an embodiment of the present invention can control the discharge amount of the content when one click is generated at the uneven protrusions 44, by adjusting the arrangement angle of the uneven protrusions 44. One click being generated at the uneven protrusions 44 may mean that movement from one uneven protrusion 44 to an adjacent uneven protrusion 44 has occurred.

For example, in the content container according to an embodiment of the present invention, the pitch of the threaded column 52 is 0.1 to 5 mm, and the uneven protrusions 44 may be arranged at regular angular intervals around the ring-shaped protrusion 43. In detail, the uneven protrusions 44 may be arranged every 0 to 30 degrees. When movement from one uneven protrusion 44 to an adjacent uneven protrusion 44 occurs, 0.01 to 0.1 mL of the content may be discharged. That is, when one click is generated at the uneven protrusions 44, 0.01 to 0.1 mL of the content may be discharged.

The uneven protrusions 44 can implement various forms for the surface that abuts against the operational feel generating part 24. The uneven protrusion 44 may comprise, based on the operating part 40, a curved inclined surface along the direction of relative rotation of the storage part 20, and a vertical surface formed at the point where the inclined surface ends. The inclined surfaces and vertical surfaces of the plurality of uneven protrusions 44 may be formed to correspond to the inclined surface and vertical surface of the locking protrusion 242 of the storage part 20.

The storage part 20 is provided with an operational feel generating part 24 having a curved inclined surface and a vertical surface in the direction of rotation, and the operating part 40 is provided with a plurality of uneven protrusions 44 having a curved inclined surface and a vertical surface in the direction of rotation. Accordingly, when the operating part 40 is rotated relative to the storage part 20, the inclined surface of the uneven protrusion 44 rotates along the inclined surface of the operational feel generating part 24, and a click is generated when the vertical surface of the uneven protrusion 44 passes over the vertical surface of the operational feel generating part 24. In addition, once the vertical surface of the uneven protrusion 44 passes over the vertical surface of the operational feel generating part 24, the two vertical surfaces face each other, so that reverse rotation of the operating part 40 can be prevented.

The center protrusion 41, the peripheral protrusion 42, and the uneven protrusions 44 are provided sequentially in the direction away from the center of the inner bottom surface of the operating part 40, and may be provided at positions overlapping each other in the height direction. Through the arrangement of these protrusions, the present embodiment can minimize the height occupied by the configurations for achieving functions such as seating of the shaft part 50 on the operating part 40 and providing an operational feel for relative rotation of the storage part 20. In addition, the present embodiment can maximize the space for storing the content in the total height of the content container 1, thereby enhancing user satisfaction.

The present embodiment includes peripheral protrusions 42 and insertion grooves 511 for seating of the shaft part 50 on the operating part 40, and includes uneven protrusions 44 and an operational feel generating part 24 for generating a click when the operating part 40 rotates relative to the storage part 20. If the peripheral protrusion 42 and insertion groove 511 are referred to as first cooperative means, and the uneven protrusion 44 and operational feel generating part 24 are referred to as second cooperative means, the first cooperative means and the second cooperative means may be arranged between the lower end of the storage part 20 and the bottom surface of the operating part 40.

If the height required to provide the first cooperative means is A and the height required to provide the second cooperative means is B, and the first and second cooperative means are arranged at different heights in the vertical direction, the portion of the operating part 40 below the storage part 20 would need to have a height of A+B or more. In this case, the content container 1 would necessarily extend downward from the storage part 20 by a height of A+B.

However, in the present embodiment, by arranging the first cooperative means and the second cooperative means on the same height, the portion of the operating part 40 below the storage part 20 can be minimized to a height of approximately A or B.

Accordingly, in the content container 1 having a function of stable rotation of the shaft part 50 and a sound-generating function when the operating part 40 rotates, the present embodiment can sufficiently secure the storage volume of the content in the content container 1 without unnecessarily increasing the overall height of the content container 1.

The seating protrusion 45 is provided in plurality and is arranged radially around the uneven protrusions 44. The seating protrusion 45 may be provided in a number and position corresponding to the peripheral protrusions 42. However, whereas the peripheral protrusion 42 protrudes upward from the bottom surface of the operating part 40, the seating protrusion 45 may protrude inward from the side wall of the operating part 40.

The lower end of the storage part 20 can engage with the inside of the plurality of seating protrusions 45, and through this, the seating protrusions 45 can achieve centering of the lower end of the storage part 20 with respect to the operating part 40. In addition, as the seating protrusion 45 interferes with the lower end of the storage part 20 in the inner-outer direction, the side wall of the storage part 20 is spaced apart from the inside of the operating part 40, so that convenience for the rotational manipulation of the operating part 40 can be improved.

In addition, with the provision of the seating protrusion 45, the operating part 40 can have improved structural strength, and the operating part 40 can be effectively prevented from being detached from the storage part 20 and the like.

The shaft part 50 is assembled to the inner bottom of the operating part 40. The shaft part 50 comprises a fixing plate 51 and a threaded column 52. The fixing plate 51 is provided in the form of a disk and can be seated on the inner bottom of the operating part 40. A plurality of insertion grooves 511 may be provided radially around the periphery of the fixing plate 51. The insertion grooves 511 of the fixing plate 51 are provided in the same number and corresponding form as the peripheral protrusions 42 of the operating part 40 and can mutually engage.

The fixing plate 51 may be provided at the base end of the threaded column 52. The threaded column 52 may be provided at the center of the fixing plate 51. In addition, the insertion grooves 511 of the fixing plate 51 engage with the peripheral protrusions 42 of the operating part 40 and, at the same time, the circumference of the fixing plate 51 can be seated inside the ring-shaped protrusion 43 of the operating part 40. Accordingly, the fixing plate 51 can be installed at an appropriate position on the bottom of the operating part 40.

The threaded column 52 has a form extending upward from the center of the fixing plate 51 and may be provided with threads (not shown). The threads of the threaded column 52 can be screw-coupled with the pressing part 60, and when the threaded column 52 rotates, rotation of the pressing part 60 is suppressed so that the pressing part 60 can move up and down along the height direction of the threaded column 52.

The threaded column 52 can engage with the center protrusion 41 provided on the inner bottom surface of the operating part 40. The threaded column 52 may have an insertion port 521 at its base end into which the center protrusion 41 is inserted, and as the center protrusion 41 is introduced into the insertion port 521, coaxiality of the shaft part 50 and the operating part 40 can be secured.

The threaded column 52 may have the form of a hollow shaft, and in order to prevent the content from entering, the upper end may be closed and the lower end may be open to form the insertion port 521.

The engagement of the insertion port 521 of the threaded column 52 and the center protrusion 41 of the operating part 40 is used to align the centers of the operating part 40 and the shaft part 50, and the mutual engagement of the circumference of the fixing plate 51 and the ring-shaped protrusion 43 of the operating part 40 is also used to align the centers of the operating part 40 and the shaft part 50. However, the insertion grooves 511 of the fixing plate 51 and the peripheral protrusions 42 of the operating part 40 may be used to suppress relative rotation of the shaft part 50 and the operating part 40. Of course, it is also possible to secure the integral rotation function of the operating part 40 and the shaft part 50 by making the shapes of the insertion port 521, the center protrusion 41, the ring-shaped protrusion 43, and the like non-circular.

The threaded column 52 may have at its end a rise restricting part (not shown) that releases engagement with the pressing part 60 so as to limit the maximum rise position of the pressing part 60. The rise restricting part is provided in a form having no threads, and can limit the maximum rise position of the pressing part 60 to a point spaced apart from the discharge part 30. Through this, the present embodiment can prevent unnecessary leakage of the content by keeping the upper surface of the pressing part 60 from directly contacting the discharge part 30.

The pressing part 60 pushes up the content by manipulation of the operating part 40. The pressing part 60 engages with the shaft part 50 and rises during relative rotation of the operating part 40 with respect to the storage part 20 so as to push up the content.

The operating part 40 and the shaft part 50 rotate integrally, and the pressing part 60 is provided non-rotatably inside the storage part 20. Since the operating part 40 rotates relative to the storage part 20, when the operating part 40 rotates, the pressing part 60 remains non-rotating.

For this purpose, the pressing part 60 may be provided with a groove 61 that engages with the uneven part 25 of the storage part 20. The storage part 20 has an uneven part 25 formed on its side wall, and the pressing part 60 may have a groove 61 having a concave curved surface shape on its circumference that engages with the convex curved surface of the uneven part 25. The form of the groove 61 may be similar to that described for the uneven part 25 above.

When the shaft part 50 is rotated by the operating part 40, rotation of the pressing part 60 can be suppressed because the groove 61 of the pressing part 60 engages with the uneven part 25 of the storage part 20. In this case, the pressing part 60 can move up and down along the threaded column 52 of the shaft part 50.

The pressing part 60 may be provided with threads 62 on its inner surface. The threads 62 of the pressing part 60 can engage with the threads of the threaded column 52, and when the operating part 40 rotates and the threaded column 52 rotates, the pressing part 60 has its rotation restricted by the storage part 20 and can move along the longitudinal direction of the threaded column 52. The threads 62 extend upward from the bottom surface of the pressing part 60, and the plurality of threads 62 and the plurality of threads of the threaded column 52 can be firmly coupled.

The pressing part 60 may be provided with threads 62 that engage with the threaded column 52 at the lower side. Through this, even if a rise restricting part is provided on the threaded column 52, the pressing part 60 can discharge the content so as to minimize the residual content. That is, the pressing part 60 can allow the content accommodated in the storage part 20 to be sufficiently discharged while not directly touching the discharge part 30.

That is, the user removes the cover part 10 from the content container 1 of the present embodiment, and then holds the operating part 40 and the discharge part 30 and rotates the operating part 40. At this time, the discharge part 30, the storage part 20, and the pressing part 60 have their rotation restricted, the operating part 40 and the shaft part 50 rotate integrally, and the pressing part 60 rises as the shaft part 50 rotates relative thereto while being screw-engaged therewith, thereby discharging the content.

The content according to an embodiment of the present invention can be accommodated in the content container 1. Referring to FIGS. 1 to 19, the content container 1 according to an embodiment of the present invention comprises: a storage part 20 that accommodates a content; a discharge part 30 that has a discharge port 31 and is coupled to the upper side of the storage part 20 so that relative rotation with respect to the storage part 20 is restricted; an operating part 40 that is rotatably assembled to the lower side of the storage part 20; a shaft part 50 that is assembled to the inner bottom of the operating part 40 so that relative rotation with respect to the operating part 40 is restricted; and a pressing part 60 that engages with the shaft part 50 and rises during relative rotation of the operating part 40 with respect to the storage part 20 so as to push up the content.

The content container 1 according to one aspect of the present invention comprises: a storage part 20 that accommodates a content; an operating part 40 that is rotatably assembled to the lower side of the storage part 20; and a pressing part 60 that pushes up the content by manipulation of the operating part 40. The content has a property of being pressurized by the pressing part 60 such that its viscosity decreases, and has a viscosity of 1,000 Pa·s to 6,000 Pa·s as measured at 70°C. The content has a hardness of 85 dyne/cm² to 500 dyne/cm² as measured at 25°C.

In addition, the content container 1 according to one aspect of the present invention comprises: a storage part 20 that accommodates a content; a shaft part 50 that rotates relative to the storage part 20; and a pressing part 60 that is screw-coupled to the shaft part 50 and pushes up the content by rotation of the shaft part 50. The content has a property of being pressurized by the pressing part 60 such that its viscosity decreases, and has a viscosity of 1,000 Pa·s to 6,000 Pa·s as measured at 70°C. The content has a hardness of 85 dyne/cm² to 500 dyne/cm² as measured at 25°C.

In addition, the content container according to one aspect of the present invention comprises: a storage part 20 that accommodates a content; a discharge part 30 that has a discharge port 31 and is coupled to the storage part 20 so as to cover the upper portion of the storage part 20; and an operating part 40 coupled to the storage part 20 so as to cover the lower portion of the storage part 20. The content has a property of having a decreased viscosity when discharged to the outside through the discharge port 31, and has a viscosity of 1,000 Pa·s to 6,000 Pa·s as measured at 70°C. The content has a hardness of 85 dyne/cm² to 500 dyne/cm² as measured at 25°C.

The storage part 20 may comprise: an uneven part 25 that is formed by being continuously recessed inward along the height direction in the side wall and limits rotation of the pressing part 60; and a protruding step 251 that is provided on the outside of the side wall and has a form that interrupts the uneven part 25 in the height direction.

The uneven part 25 may have a curved surface shape that is convex inward, and the pressing part 60
may have a groove having a concave curved surface shape on its circumference that engages with the uneven part 25.

The content container may further comprise a cover part 10 having a stopper protrusion 11 that is inserted into the discharge port 31, wherein the stopper protrusion 11 has a height such that it is partially inserted into the discharge port 31 and is formed in a hollow form.

The discharge part 30 may surround the outer circumference of the upper end of the storage part 20 and be coupled to the storage part 20 so that relative rotation is restricted, and may comprise a sealing rib 34 that protrudes downward and engages with the inner circumference of the upper end of the storage part 20.

The storage part 20 may further comprise a rim 22 provided so as to protrude on the outside of the side wall, and the discharge part 30 may further comprise a ring-shaped groove 32 that engages with the rim 22 so as to maintain coupling with the storage part 20.

The sealing rib 34 may be provided with a sealing protrusion 341 on its outer circumference that abuts against the inner circumference of the upper end of the storage part 20, and the sealing protrusion 341 can block the gap between the sealing rib 34 and the upper end of the storage part 20 so as to block leakage of the content.

In the content container 1, leakage of the content may occur at the portions where the pressing part 60 and the storage part 20, the pressing part 60 and the shaft part 50, the shaft part 50 and the storage part 20, the storage part 20 and the discharge part 30, and the cover part 10 and the discharge port 31, and the like, are coupled. Accordingly, the content container 1 according to an embodiment of the present invention can increase the effect of preventing leakage of the content through the structure of the protruding step 251 and the like.

However, even in such a content container 1, leakage of the content may occur depending on the viscosity and the like of the content. That is, the leakage prevention effect of the content container 1 can be further enhanced by controlling the content. In detail, a content having viscosity and the like exhibiting a leakage prevention effect in the content container 1 can be injected into the content container 1. Accordingly, the content and the content container 1 can exhibit a synergistic effect in preventing leakage.

Hereinafter, the content will be described in detail.

The content can have a viscosity that prevents leakage inside the content container 1. In particular, the content can maintain a viscosity of at least a certain value even at relatively high temperatures. That is, the content can have a lower limit value of viscosity under a certain temperature condition.

In order to increase the viscosity of the content, the content comprises a gelling agent. The gelling agent can convert an oily component into a gel phase or solidify the oily component. The content may comprise 3 to 20% by weight of the gelling agent based on the total weight of the content. Preferably, the content may comprise 5 to 15% by weight of the gelling agent based on the total weight of the content. Within the range of the gelling agent, the viscosity and hardness of the content can be maintained within a certain range. In addition, within the range of the gelling agent, leakage of the content can be prevented. In addition, within the range of the gelling agent, the content can exhibit excellent usability.

The gelling agent is not particularly limited as long as it can convert an oily component into a gel phase or solidify the oily component, and may be used alone or in combination. The gelling agent may be an oil thickening gelling agent. The gelling agent may be one having excellent compatibility with oils such as hydrocarbons, esters, triglycerides, and isoparaffins.

The gelling agent may comprise, for example, dextrin fatty acid esters, sucrose fatty acid esters, inulin fatty acid esters, and the like. The strength of the gel of the content may vary depending on the acyl group of the dextrin fatty acid ester. Preferably, the gelling agent may be a dextrin fatty acid ester.

The dextrin fatty acid ester may comprise dextrin palmitate, dextrin palmitate/ethylhexanoate, dextrin myristate, and the like. Preferably, the dextrin fatty acid ester may be dextrin palmitate.

The dextrin palmitate has excellent compatibility with oils such as hydrocarbons, esters, triglycerides, and isoparaffins. In addition, the dextrin palmitate can maintain the structural viscosity of the content at high temperatures compared to wax.

The content may require different viscosities when stored in the content container 1 and when discharged from the content container 1 for use. When the content is stored, it may be advantageous to have a high viscosity to prevent leakage, but when the content is easily discharged along the discharge part 30 and can be spread evenly and widely on the skin or the like, it may be advantageous for the content to have a relatively low viscosity. Accordingly, it is preferable that the viscosity is adjusted according to the situation.

For viscosity adjustment according to the situation, the content has thixotropy. The thixotropy refers to a property in which the flowability of a fluid changes due to an external force such as friction or shear force. The thixotropy can also be expressed as a property in which the viscosity and the like of a fluid changes depending on an external force. By controlling the thixotropy, the degree to which the viscosity, hardness, and the like of the content change with external force can be controlled.

Controlling the viscosity of the content through thixotropy is preferable in terms of storing and using the content. For example, it is preferable that the viscosity of the content is high to prevent the content from leaking to the outside of the container when the content is stored in the container and no external force acts on the content. Conversely, when an external force acts on the container and the content is used, it is preferable that the viscosity of the content is low so that the content is easily discharged and easily applied to the skin or the like. In addition, if the viscosity is high when the content is used, the user may feel a sticky and heavy feel when applied.

A thixotropic agent may be added to the content to control the thixotropy of the content. The thixotropic agent can control bonding between particles contained in the content. In detail, the thixotropic agent can form a network structure between particles (or molecules) contained in the content. The content can have structural viscosity due to the network structure. The structural viscosity may have the effect of increasing the viscosity of the content before external force is applied, and when external force is applied, the network structure may be broken by the external force and the viscosity of the content may decrease.

The content comprises a thickener. The thickener can control the thixotropy of the content. That is, the thickener can exhibit the effect of a thixotropic agent. The content can have structural viscosity due to the thickener. It is preferable that the thickener can make the structural viscosity of the content relatively high.

The thickener may have a powder form for uniform dispersion. The thickener may have an average particle size of 1 nm to 100 µm. The thickener can be dispersed in the content to induce secondary inter-particle bonding in the content so that the content has structural viscosity.

The content may comprise 0.01 to 10% by weight of the thickener based on the total weight of the content. Preferably, the content may comprise 0.1 to 5% by weight of the thickener based on the total weight of the content. Within the range of the thickener, the viscosity and hardness of the content can be maintained within a certain range. In addition, within the range of the thickener, leakage of the content can be prevented. In addition, within the range of the thickener, the spreadability of the content is not deteriorated and excellent usability and excellent storage stability can be exhibited.

The thickener is not particularly limited as long as it can impart thixotropy to the content, and may be used alone or in combination. The thickener may comprise, for example, one or more selected from the group consisting of silica silylate and hectorite-based thickeners.

The silica silylate may have an average particle size of 1 to 100 nm, and the hectorite-based thickener may have an average particle size of 1 to 100 µm.

The silica silylate may be one in which the -OH group on the surface of silica has been functionalized with a methylsilyl group through a reaction. Silica silylate may comprise silica dimethyl silylate or silica trimethyl silylate. Preferably, the silica silylate may be silica dimethyl silylate. The silica silylate may be hydrophobic.

The silica silylate may be produced by reacting fumed silica. The fumed silica has a large specific surface area, is excellent in oil absorption capacity, and has excellent thickening properties.

The hectorite-based thickener may comprise hectorite, disteardimonium hectorite, quaternium-18 hectorite, and stearalkonium hectorite. Preferably, the hectorite-based thickener may be disteardimonium hectorite. The disteardimonium hectorite can increase the structural viscosity of the content while maintaining the spreadability of the content.

The content may comprise an oily component forming an oil phase part. The oily component may provide the content with smooth spreadability, block evaporation of moisture from the skin or the like to increase moisturizing power, or increase adhesion to the skin or the like.

The oily component may comprise one or more selected from the group consisting of hydrogenated polyisobutene, pentaerythritol fatty acid esters, phytosterol fatty acid esters, diisostearyl malate, and silicone oil.

Here, the hydrogenated polyisobutene, pentaerythritol fatty acid ester, and phytosterol fatty acid ester may be binders. The binder may be a high-viscosity binder. The binder may have a viscosity of 10,000 mm²/s or more. The binder can prevent leakage of the content.

The diisostearyl malate and silicone oil may be emollients that improve skin moisture and flexibility. The emollient may have a relatively lower viscosity than the binder.

Commercially available hydrogenated polyisobutene products include Parleam (manufactured by Nippon Oil Fats), Panalane H-300 E (MW = 1340 g/mol) (manufactured by Amoco), Viseal 20000 (MW = 6000 g/mol) (manufactured by Synteal), Rewopal PIB 1000 (MW = 1000 g/mol) (manufactured by Witco), and the like.

The silicone oil may comprise, for example, cyclopentasiloxane, methyl trimethicone, caprylyl methicone, cyclomethicone, cyclotetrasiloxane, cyclohexasiloxane, cycloheptasiloxane, decamethylcyclopentasiloxane, cyclotrisiloxane, dimethicone, caprylyl trimethicone, cetearyl methicone, hexadecyl methicone, hexyl methicone, lauryl methicone, myristyl methicone, phenyl methicone, stearyl methicone, stearyl dimethicone, trifluoropropyl dimethicone, cetyl dimethicone, polyphenyl methylsiloxane, polydimethylsiloxane, methylphenyl polysiloxane, methyl trimethicone, diphenylsiloxy phenyl trimethicone, phenyl trimethicone, diphenyl dimethicone, and the like. Preferably, the silicone oil may be caprylyl methicone.

The pentaerythritol fatty acid ester may comprise, for example, dipentaerythrityl tetrahydroxystearate/tetraisostearate, pentaerythrityl tetraisostearate, and dipentaerythrityl hexahydroxystearate/hexastearate/hexarosinate. Preferably, the pentaerythritol fatty acid ester may be dipentaerythrityl hexahydroxystearate/hexastearate/hexarosinate. Commercially available pentaerythritol fatty acid ester products include COSMOL 168M, COSMOL 168E, COSMOL 168AR (all manufactured by THE NISSHIN OILLIO GROUP, LTD.), and the like.

The phytosterol fatty acid ester may comprise, for example, phytosteryl oleate, dimer dilinoleic acid dimer dilinoleyl bis(behenyl/isostearyl/phytosteryl), dimer dilinoleic acid (phytosteryl/isostearyl/cetyl/stearyl/behenyl), and macadamia nut fatty acid phytosteryl. Preferably, the phytosterol fatty acid ester may be phytosteryl/isostearyl/cetyl/stearyl/behenyl dimer dilinoleate. Commercially available phytosterol fatty acid ester products include PLANDOOL-G and PLANDOOL-H (both manufactured by NIPPON FINE CHEMICAL CO., LTD.).

The content may comprise 50 to 89% by weight of the oily component based on the total weight of the content. The oily component may comprise 10 to 79% by weight of binder based on the total weight of the content. Preferably, the oily component may comprise 10 to 49% by weight of binder based on the total weight of the content.

The oily component may comprise 40 to 79% by weight of emollient based on the total weight of the content. Within the range of the oily component, leakage of the content can be prevented. In addition, the content can exhibit excellent flexibility, usability, and adhesion.

The content may comprise an emulsifier in order to increase the stability of the content. The emulsifier may comprise one or more selected from silicone-based emulsifiers, stearate-based emulsifiers, and sorbitan-based emulsifiers.

The silicone-based emulsifier may comprise one or more selected from the group consisting of cetyl PEG/PPG-10/1 dimethicone, PEG-10 dimethicone, lauryl PEG-9 polydimethylsiloxy ethyl dimethicone, and lauryl polyglyceryl-3 polydimethylsiloxy ethyl dimethicone.

The stearate-based emulsifier may be one or more selected from the group consisting of PEG-30 dipolyhydroxystearate, glyceryl stearate, PEG-100 stearate, triisostearate, and polyoxyethylene diisostearate, but is not limited thereto.

The sorbitan-based emulsifier may be one or more selected from the group consisting of sorbitan olivate, sorbitan stearate, sorbitan isostearate, sorbitan tristearate, sorbitan sesquistearate, sorbitan palmitate, sorbitan laurate, sorbitan oleate, sorbitan sesquioleate, sorbitan trioleate, sorbitan stearate/sorbityl laurate, polysorbate 20, polysorbate 40, polysorbate 60, polysorbate 65, polysorbate 80, and polysorbate 85, but is not limited thereto.

Preferably, the emulsifier may be a sorbitan-based emulsifier. Preferably, the sorbitan-based emulsifier may comprise at least one of sorbitan olivate and sorbitan stearate.

The content may comprise 0.1 to 10% by weight of the emulsifier based on the total weight of the content. Preferably, the content may comprise 1 to 8% by weight of the emulsifier, and more preferably 2 to 7% by weight.

The content may comprise an aqueous component. The aqueous component may refer to a water phase component, i.e., a component comprising purified water. The aqueous component may further comprise a polyol. For example, the polyol may be one or more selected from the group consisting of 1,2-hexanediol, glycerin, 1,3-butylene glycol, dipropylene glycol, and propylene glycol. The content may comprise 5 to 30% by weight of the aqueous component based on the total weight of the content. Preferably, the content may comprise 5 to 10% by weight of the aqueous component based on the total weight of the content.

The content according to an embodiment of the present invention may further comprise additives such as colorants, pigments, moisturizing agents, surfactants, fragrances, fillers, preservatives, and neutralizing agents without limitation.

The content may have a solid or semi-solid form at room temperature. For example, the semi-solid form may be a cream form, gel form, or paste form. The content may undergo a phase change to a phase with good flowability when subjected to an external force, or the viscosity may decrease.

In addition, the content can maintain the viscosity within a certain range to prevent leakage. Accordingly, the content can maintain the viscosity within a certain range even at high temperatures. The content may have a viscosity of 1,000 Pa·s to 6,000 Pa·s as measured at 70°C. Within the viscosity range, leakage of the content can be prevented.

In addition, the content may have thixotropy. For example, the content can have a decreased viscosity when pressurized and subjected to an external force. In order to prevent leakage even when the content is pressurized in this way, the content can maintain the viscosity within a certain range.

The threaded column 52 of the shaft part 50 and the threads 62 of the pressing part 60 overlap and are coupled, and the coupling length of the shaft part 50 and the pressing part 60 is longer than the coupling length between the storage part 20 and the discharge part 30. Accordingly, the possibility of leakage at the shaft part 50 and the pressing part 60 is lower compared to the storage part 20 and the discharge part 30.

Within the viscosity range, the content can prevent leakage of the content between the storage part 20 and the discharge part 30. In addition, within the viscosity range, the content can prevent leakage of the content between the shaft part 50 and the pressing part 60. Preferably, within the viscosity range, the content can prevent leakage of the content even between the storage part 20 and the discharge part 30, where the possibility of leakage is relatively high.

The content can maintain the hardness within a certain range. The content may have a hardness of 85 dyne/cm² to 500 dyne/cm² as measured at 25°C. The hardness may be the hardness of the content before it is discharged from the content container.

When the hardness is low, the components of the content are not firmly bonded to each other, components with low compatibility in the content may separate, and oil sweating may occur.

However, since the gelling agent of the content forms a dense and tight lattice structure, components such as the oily component contained in the content can be fixed in the lattice structure. Accordingly, the content can have excellent stability without leaking between the storage part 20 and the discharge part 30 even when the content container is tilted. The content may have a decreased hardness when discharged.

In detail, within the hardness range, the content can prevent leakage of the content between the storage part 20 and the discharge part 30. In addition, within the hardness range, the content can prevent leakage of the content between the shaft part 50 and the pressing part 60. Preferably, within the hardness range, the content can prevent leakage of the content even between the storage part 20 and the discharge part 30, where the possibility of leakage is relatively high.

After the content is discharged from the content container, the hardness of the content can be smaller than before discharge. For example, the hardness of the content after being discharged from the content container as measured at 25°C may be 40 to 70% of the hardness before discharge.

Hereinafter, the present invention will be described in more detail through experimental examples. The technical scope of the present invention is not limited by the following examples and comparative examples.

### Experimental Example 1: Evaluation of Leakage Stability and Feel of Use According to Gelling Agent or Thickener

### Examples 1 to 4 and Comparative Example 1

Contents of Examples 1 to 4 and Comparative Example 1 were prepared with the compositions shown in Table 1 below (unit: % by weight).

Specifically, the preparation method of the content is as follows.
1) The oil phase was heated to 85°C.
2) The gelling agent, thickener, and emulsifier were added to the oil phase of step 1), and the thickener was dispersed for 10 minutes at 5,000 rpm using a homogenizer.
3) The aqueous phase was dissolved while mixing with a disperser.
4) The aqueous phase mixture of step 3) was mixed with the oil phase mixture of step 2), and an emulsion was prepared for 10 minutes at 70°C and 5,000 rpm using a homogenizer.
5) Thereafter, the emulsion was fully stirred, deaerated, and then allowed to cool naturally.

**Table 1**

| Component | Ingredient | Ex. 1 | Ex. 2 | Ex. 3 | Ex. 4 | Comp. Ex. 1 |
|---|---|---|---|---|---|---|
| Gelling agent | Dextrin Palmitate | 8.50% | 8.50% | 8.50% | 8.50% | 0 |
| | Microcrystalline wax | 0 | 0 | 0 | 0 | 8.50% |
| Oil phase | Hydrogenated Polyisobutene | 25% | 25% | 25% | 25% | 25% |
| | Diisostearyl Malate, Caprylyl Methicone | 50-60% | 50-60% | 50-60% | 50-60% | 50-60% |
| Emulsifier | Sorbitan Olivate, Sorbitan Stearate | 5% | 5% | 5% | 5% | 5% |
| Thickener | Disteardimonium Hectorite | 0 | 0 | 1% | 2% | 1% |
| | Silica Dimethyl Silylate | 1% | 2% | 0 | 0 | 0 |
| Aqueous phase | Water, 1,2-Hexanediol, Glycerin | 5-10% | 5-10% | 5-10% | 5-10% | 5-10% |

As shown in Table 2 below, the viscosity and hardness of the contents of Examples 1 to 4 and Comparative Example 1 were measured according to the gelling agent and thickener, and the feel of use and frequency of leakage were confirmed.

Specifically, the viscosity was measured using a Brookfield DV2TLVTJ0 viscometer equipped with spindle LV-04 spindle 64 and a Model G laboratory stand (unit: Pa·s). After completely melting the content by heating to above 80°C using a microwave, 80 mL of the content was placed in a PYREX^{®} Griffin Low Form 100 mL Beaker, Graduated, and sampling was performed while slowly stirring and adjusting to 70°C. The speed of the Brookfield DV2TLVTJ0 viscometer was set to 30 rpm, the end condition was set to 1 minute, and the viscosity was measured.

The hardness was measured using a FUDOH RHEO METER RTC-3005D from RHEOTECH, equipped with spindle No. 3, 10ø (unit: dyne/cm²). After completely melting the content by heating to above 80°C using a microwave, 10 mL of the content adjusted to 70°C was placed in an aluminum square dish (X-1) and sampling was performed while storing in a constant temperature chamber at 25°C for 1 hour. Using the FUDOH RHEO METER RTC-3005D, the hardness was measured based on the force-time curve up to 2 mm from the surface of the content. The spindle used for the hardness measurement was No. 3, 10ø, the table speed was 2.0 cm/min, and the P (peak hold: hold at maximum stress) maximum load was 5 N.

The feel of use was evaluated by sensory evaluation on 20 women aged 20 to 40 who applied an appropriate amount (0.03 g) to their lips and rated their satisfaction with uniform spreadability, a light non-sticky feel of use, and the like according to the following evaluation criteria.

Evaluation criteria: 4-5 points: ⊚, 3-4 points: ∘, 2-3 points: △, 2 points or less: X

The frequency of leakage was evaluated as follows: 15 mL of content was filled into the content container and allowed to cool naturally; after a stabilization period of 16 hours or more, the content container was kept upside down (inverted) in a constant temperature chamber maintained at 50°C; and the content container was used repeatedly every day for evaluation.

Specifically, each day the content container was taken out of the 50°C constant temperature chamber, stored at room temperature for 1 hour, the cover part was opened to check the leakage state, 0.15 g of the content was discharged and wiped off, and the content container was put back into the 50°C constant temperature chamber for storage. The discharge and storage process of the content was repeated for 21 days (3 weeks), and it was evaluated how many of a total of 5 content container samples had leakage. The presence or absence of leakage was confirmed at the lower end position of the discharge part in close contact with the storage part and at the upper end position of the operating part in close contact with the auxiliary coupling part.

**Table 2**

| | Ex. 1 | Ex. 2 | Ex. 3 | Ex. 4 | Comp. Ex. 1 |
|---|---|---|---|---|---|
| Viscosity (70°C) | 1280 | 3920 | 2560 | 4380 | 800 |
| Hardness (25°C) | 122.5 | 144.5 | 132.5 | 101 | 120 |
| Feel of use | ○ | ○ | ○ | ○ | Δ |
| Frequency of leakage (50°C) | 0/5 | 0/5 | 0/5 | 0/5 | 4/5 |

As shown in Examples 1 to 4, the high-temperature viscosity increased in proportion to the content of the thickener. Accordingly, the contents of Examples 1 to 4 did not leak from the container even at 50°C, which is higher than room temperature. Comparing Example 3 and Comparative Example 1, leakage was prevented in Example 3 compared to Comparative Example 1 where leakage occurred.

Meanwhile, the contents of Examples 1 to 4 can have a solid or semi-solid form at room temperature. The contents of Examples 1 to 4 can have a hardness of at least a certain value under room temperature conditions.

The contents of Examples 1 to 4 may undergo a phase change to a phase with excellent flowability or the viscosity may decrease due to thixotropy when subjected to an external force or when the temperature increases. The fact that the contents of Examples 1 to 4 have a viscosity within a certain range at 70°C supports the finding that the contents have a viscosity within a certain range even when they are pressurized during use. Accordingly, the content may not leak from the content container even if it is pressurized during use.

In addition, since the contents of Examples 1 to 4 have a hardness within a certain range, the oily component is not separated from the content, and excellent formulation stability and storage stability can be achieved.

### Experimental Example 2: Evaluation of Leakage Stability and Feel of Use According to Components Contained in the Oil Phase

Contents of Examples 3, 5, 6, and Comparative Example 2 were prepared with the compositions shown in Table 3 below (unit: % by weight). Examples 3, 5, 6, and Comparative Example 2 were prepared in the same manner as in Experimental Example 1.

**Table 3**

| Component | Ingredient | Ex.3 | Ex.5 | Ex.6 | Comp. Ex. 2 |
|---|---|---|---|---|---|
| Gelling agent | Dextrin Palmitate | 8.50 % | 8.50 % | 8.50 % | 8.50 % |
| Oil | Hydrogenated Polyisobutene | 25% | 0 | 0 | 0 |

| | | | | | |
|---|---|---|---|---|---|
| phase | PHYTOSTERYL/ISOSTEARYL/CETYL/STEAR YL/BEHENYL DIMER DILINOLEATE (PLANDOOL-H) | 0 | 25% | 0 | 0 |
| | DIPENTAERYTHRITYL HEXAHYDROXYSTEARATE/HEXASTEARAT E/HEXAROSINATE (COSMOL 168ARV) | 0 | 0 | 25% | 0 |
| | BIS-DIGLYCERYL POLYACYLADIPATE-2 (SOFTISAN 649TN) | 0 | 0 | 0 | 25% |
| | Diisostearyl Malate, Caprylyl Methicone | 50-60% | 50-60% | 50-60% | **50-**60% |
| Emulsifier | Sorbitan Olivate, Sorbitan Stearate | 5% | 5% | 5% | 5% |
| Thickener | Disteardimonium Hectorite | 1% | 1% | 1% | 1% |
| Aqueous phase | Water, 1,2-Hexanediol, Glycerin | 5-10% | 5-10% | 5-10% | 5-10% |

As shown in Table 4 below, the viscosity and hardness of the contents of Examples 3, 5, 6, and Comparative Example 2 were measured according to the oil phase, and the feel of use was confirmed. Viscosity measurement and the like were performed in the same manner as in Experimental Example 1, except that the frequency of leakage was measured over 14 days (2 weeks).

**Table 4**

| | Ex. 3 | Ex. 5 | Ex. 6 | Comp. Ex. 2 |
|---|---|---|---|---|
| Viscosity (70°C) | 2560 | 3160 | 1020 | 1000 |
| Hardness (25°C) | 132.5 | 136.5 | 121 | 59.5 |
| Feel of use | ○ | ⊚ | ○ | ⊚ |
| Frequency of leakage (50°C) | 0/5 | 0/5 | 0/5 | 3/5 |

In Experimental Example 2, an oil having a higher viscosity than the emollient was added to the oil phase in order to reduce the leakage risk of the content. Here, Examples 3 and 5 comprising hydrogenated polyisobutene and phytosterol fatty acid ester had relatively higher high-temperature viscosity and hardness compared to Comparative Example 2 comprising bis-diglyceryl polyacyladipate-2.

While leakage of the content occurred 3 times in Comparative Example 2, no leakage occurred in Examples 3 and 5 and Example 6 comprising pentaerythritol fatty acid ester. It was confirmed that the viscosity was increased by the hydrogenated polyisobutene and phytosterol fatty acid ester and pentaerythritol fatty acid ester.

In addition, hydrogenated polyisobutene and phytosterol fatty acid ester and pentaerythritol fatty acid ester can increase the hardness in combination with the gelling agent, dextrin palmitate. Due to the increased hardness, the oily component is not separated from the content, and excellent formulation stability and storage stability can be achieved. Accordingly, hydrogenated polyisobutene and phytosterol fatty acid ester and pentaerythritol fatty acid ester can increase the storage stability of the content.

### Experimental Example 3: Evaluation of Change in Hardness Before and After Discharge

**Table 5**

| | Before discharge | After discharge |
|---|---|---|
| Hardness (25°C) | 132.5 | 60.5 |

The hardness of Example 3 before and after discharge from the content container was confirmed. The hardness of Example 3 after discharge was measured immediately after Example 3 was discharged from the content container, using the same method as the method for measuring hardness in the above experiment, and the measurement was performed while the operational feel generating part of the operating part continuously interacted with 1 to 3 uneven protrusions during rotation. As shown in Table 5, the hardness of the content became smaller after discharge than before discharge. The hardness of Example 3 decreased to approximately 45% compared to before discharge.

As shown in Table 5, the content decreases in hardness as it is discharged, but the content can be stably stored without leaking from the content container. In particular, no leakage occurred between the discharge part and the storage part of the content container.

### Experimental Example 4: Evaluation of Discharge Amount in Content Container

The discharge amount of Example 3 was evaluated when the operating part in the content container rotated by the angle between adjacent uneven protrusions. In the content container, the angle between adjacent uneven protrusions was 18 degrees, and the pitch of the threaded column was 1.2 mm. At this time, when the operating part rotated between adjacent uneven protrusions, 0.035 mL of the content was discharged. The amount of content may increase in proportion to the degree of rotation of the operating part.

When the operating part rotates between adjacent uneven protrusions, the uneven protrusions and the operational feel generating part collide, generating a clicking sound or a tactile catching sensation. In this way, the uneven protrusions and the operational feel generating part can provide an operational feel to the content container. In addition, since a certain amount of content is discharged when an operational feel is generated, the user can control the amount of content based on sensory feel alone.

Although the present invention has been described above with a focus on the embodiments of the present invention, this is merely an example and does not limit the present invention. Those skilled in the art will understand that various combinations, modifications, and applications not illustrated in the embodiments are possible without departing from the essential technical content of the present embodiment. Accordingly, technical content related to modifications and applications that can be easily derived from the embodiments of the present invention should be interpreted as being included in the present invention.

## Claims

1. A content container comprising:
a storage part that accommodates a content;
a shaft part that rotates relative to the storage part; and
a pressing part that is screw-coupled to the shaft part and pushes up the content by rotation of the shaft part,
wherein the content has a viscosity of 1,000 Pa·s to 6,000 Pa·s as measured at 70°C.

2. The content container according to claim 1, wherein the content has a hardness of 85 dyne/cm² to 500 dyne/cm² as measured at 25°C.

3. The content container according to claim 1, wherein the content comprises:
5 to 15% by weight of a gelling agent based on the total weight of the content and
0.1 to 5% by weight of a thickener based on the total weight of the content.

4. The content container according to claim 3, wherein the gelling agent comprises a dextrin fatty acid ester.

5. The content container according to claim 3, wherein the thickener comprises one or more selected from the group consisting of silica silylate and a hectorite-based thickener.

6. The content container according to claim 1, wherein the content comprises one or more oily components selected from the group consisting of hydrogenated polyisobutene, pentaerythritol fatty acid esters, phytosterol fatty acid esters, diisostearyl malate, and silicone oil.

7. The content container according to claim 6, comprising 50 to 89% by weight of the oily component based on the total weight of the content.

8. A content container comprising:
a storage part that accommodates a content;
a discharge part that has a discharge port and is coupled to an upper side of the storage part;
an operating part that is rotatably assembled to a lower side of the storage part; and
a pressing part that pushes up the content by manipulation of the operating part,
wherein the discharge part has a form that engages with both an outer circumference and an inner circumference at the upper end of the storage part, and
the operating part has an uneven structure between the operating part and the storage part.

9. The content container according to claim 8, wherein the storage part comprises:
an uneven part that is formed by being continuously recessed inward along the height direction in the side wall and limits rotation of the pressing part; and
a protruding step that is provided on the outside of the side wall and has a form that interrupts the uneven part in the height direction.

10. The content container according to claim 9,
wherein the uneven part has a curved surface shape that is convex inward, and
the pressing part has a groove having a concave curved surface shape on its circumference that engages with the uneven part.

11. The content container according to claim 9, further comprising:
a cover part having a stopper protrusion that is inserted into the discharge port,
wherein the stopper protrusion has a height such that it is partially inserted into the discharge port and is formed in a hollow form.

12. The content container according to claim 9,
wherein the storage part further comprises a rim provided so as to protrude on the outside of the side wall, and
the discharge part further comprises a ring-shaped groove that engages with the rim so as to maintain coupling with the storage part.

13. The content container according to claim 9,
wherein the discharge part surrounds the outer circumference of the upper end of the storage part and is coupled to the storage part so that relative rotation is restricted, and comprises a sealing rib that protrudes downward and engages with the inner circumference of the upper end of the storage part.

14. The content container according to claim 13,
wherein the sealing rib is provided with a sealing protrusion on its outer circumference that abuts against the inner circumference of the upper end of the storage part, and
the sealing protrusion blocks the gap between the sealing rib and the upper end of the storage part so as to block leakage of the content.

15. A content container comprising:
a storage part that accommodates a content;
an operating part that is rotatably assembled to a lower side of the storage part; and
a pressing part that pushes up the content by manipulation of the operating part
wherein the operating part comprises a plurality of uneven protrusions provided radially, and
the storage part comprises an operational feel generating part that cooperates with the uneven protrusions to provide an operational feel relating to a rotation angle of the operating part and an extent of upward and downward movement of the pressing part.

16. The content container according to claim 15, wherein when the operating part rotates by a rotation angle between adjacent uneven protrusions, 0.01 to 0.1 mL of the content is discharged.

17. The content container according to claim 15, wherein the uneven protrusions are arranged at regular angular intervals along the direction of rotation of the operating part.

18. The content container according to claim 15, further comprising:
a shaft part having a threaded column formed on its side surface and rotating relative to the storage part,
wherein the threaded column has a pitch of 0.1 to 5 mm.

19. The content container according to claim 15, wherein the operational feel generating part comprises:
an arm that extends horizontally in a cantilever form from the lower end of the storage part; and
a locking protrusion that protrudes downward from the arm.

20. The content container according to claim 15, wherein the operating part further comprises:
a center protrusion provided at the center of the inner bottom surface; and
a ring-shaped protrusion provided around the center protrusion to guide relative rotation of the storage part with respect to the operating part,
wherein the uneven protrusions are provided around the ring-shaped protrusion.
